Europäisches Patentamt

European Patent Office

Office européen des brevets

(11) Publication number: **0 112 233**
**A1**

(12) # EUROPEAN PATENT APPLICATION

(21) Application number: **83402335.0**

(22) Date of filing: **05.12.83**

(51) Int. Cl.³: **A 23 K 1/17**

(30) Priority: **13.12.82 US 449086**

(43) Date of publication of application:
**27.06.84 Bulletin 84/26**

(84) Designated Contracting States:
**CH DE FR GB IT LI NL**

(71) Applicant: MERCK & CO. INC.
126, East Lincoln Avenue P.O. Box 2000
Rahway New Jersey 07065(US)

(72) Inventor: Pellegrino, Ronald M.
176 Linden Street
Bridgewater New Jersey 08807(US)

(74) Representative: Warcoin, Jacques et al,
Cabinet Régimbeau 26, avenue Kléber
F-75116 Paris(FR)

(54) Berninamycin a as a growth permittant.

(57) Berninamycin A, a known antibiotic, is disclosed as being agent for permitting the growth of monogastric meat producing animals, in particular, chickens and swine. Composition for such use are also disclosed.

EP 0 112 233 A1

16838

TITLE OF THE INVENTION
BERNINAMYCIN A AS A GROWTH PERMITTANT

BACKGROUND OF THE INVENTION

Berninamycin A is a known sulfur containing modified peptide antibiotic agent disclosed in U.S. Patent 3,689,639 issued 5 September 1972 to Bergy et al. Berninamycin A is isolated from the fermentation of a broth of Streptomyces bernensis Dietz sp. nov. The compound is disclosed as being particularly active in inhibiting the growth of Bacillus subtilis and thus controlling the growth of slime and corrosion in petroleum products caused by such bacterium.

SUMMARY OF THE INVENTION

This invention is concerned with the use of Berninamycin A as a growth permittant in monogastric meat producing animals, particularly chickens and swine, thus it is an object of this invention to describe such uses and it is a further object to describe the conditions and results obtained by the use of Berninamycin A on the growth of chickens. A further object is to describe the compositions containing Berninamycin A for such uses. Additional objects will become apparent from a reading of the following description.

DESCRIPTION OF THE INVENTION

Berninamycin A has been shown to be an effective growth permittant in monogastric meat producing animals, particularly chickens and swine. As a result when Berninamycin A is fed to such animals they are seen to have an increase in weight over that which would normally be expected, and in addition, the amount of feed required to produce a unit of weight gain is reduced when compared with unmedicated controls and also with positive controls fed other growth permitting agents.

For example, when Berninamycin A is fed to chickens for nine days at a level of ten or 20 parts per million (ppm) in feed, the chickens are seen to have a relative weight gain increase of +24.4 and +27.9% respectively, when compared with chickens fed the unmedicated feed. In addition, the ratio of the feed intake to the weight gain is seen to be -16.0 and -16.6% respectively, indicating that the chickens fed Berninamycin A achieved their weight gain using at least 16% less feed. These data demonstrate a substantial growth permitting effect and indicate that Berninamycin A is a highly effective agent for growth permittancy in chickens.

Furthermore, Berninamycin A is observed to have a very low level of absorption in the animal, thus tissue residues and withdrawal times for animals fed Berninamycin A medicated feeds should be minimal.

When the Berninamycin A is used as a growth permitter in animals it can be administered as a component of the feed of the animals or may be dissolved or suspended in the drinking water.

When the Berninamycin A is used as a component of animal feed it is first formulated as a feed supplement. In such feed supplements Berninamycin A is present in relatively concentrated amounts intimately dispersed in an inert carrier or diluent. The feed supplement can be added directly to the feed or made into a premix by intermediate dilution or blending steps. By inert carrier is meant one that will not react with the Berninamycin A and one that may be administered safely to animals. Preferably the carrier is one that is or may be an ingredient of the animal feed ration. Typical carriers are diluents suitable for such compositions include for example, distillers dried grains, corn meal, citrus meal, fermentation residues and ground oyster shells, wheat shorts, molasses solubles, corn cob meal, edible bean mill feed soya grits, crushed limestone and the like. The antibiotic is intimately dispersed throughout the carrier by methods such as grinding, stirring, milling or tumbling.

Compositions containing from about 5 to 50% by weight of the antibiotic are particular suitable as feed supplements.

Examples of typical feed supplements containing Berninamycin A dispersed in a solid carrier are:

TABLE

|                |                        | Lbs. |
|----------------|------------------------|------|
| Formulation A  | Berninamycin A         | 5    |
|                | Wheat Standard Middling | 95   |
| Formulation B  | Berninamycin A         | 50   |
|                | Corn Distillers Grains | 50   |

These and similar feed supplements are prepared by uniformly mixing the antibiotic with the carrier.

The feed supplement can be added directly to the feed or made into a premix by an intermediate dilution or blending step with an orally ingestible carrier. Compositions containing 0.03% to 5% by weight of the antibiotic are particularly suitable as premixes. These premixes are prepared by uniformly mixing the antibiotic with an orally ingestible carrier.

Such supplements or premixes are added to the animal feed in an amount to give the finished feed the concentratiion of Berninamycin A desired for growth permittancy. In chickens Berninamycin A is fed a final concentration of between 1 and 100 ppm, preferably between 10 and 20 ppm of feed in order to achieve the desired growth permitting result.  In the case of swine,
Berninamycin A may be administered in the feed at
similar levels.

In the above discussion of this invention
emphasis has been placed on solid compositions
wherein the Berninamycin A is mixed with an edible
carrier in a feed supplement in a so-called premix or
in the final feedstuff.  This is the preferred method
of administering the Berninamycin A.  However, an
alternate method is to dissolve or suspend the
Berninamycin A in the drinking water of the animals.
The quantity that may be suspended in the water
without undue settling is limited.  Emulsifiers or
surface active agents may be employed for this letter
purpose.

It will be also understood by those skilled
in this art that special feed supplment formulations
and finished animal feeds containing Berninamycin A
may also include vitamins, other antibiotics and
growth promoting and permitting agents and other
nutritional substances.

IN THE CLAIMS:

1.  An oral composition for permitting increased growth in monogastric meat producing animals which comprises an amount of Berninamycin A effective for permitting such growth, and a non-toxic pharmaceutically acceptable carrier.

2.  A composition of Claim 1 wherein the monogastric meat producing animals are chickens or swine.

3.  The composition of Claim 2 which is the animal's medicated feed or medicated drinking water.

4.  A composition of Claim 3 which is the animal's medicated feed containing from 50-300 grams of Berninamycin A per ton of medicated feed.

5.  A composition of Claim 2 which is a feed supplement.

6.  A composition of Claim 5 in which the feed supplement contains from 5 to 50 percent of Berninamycin A.

7.  A method for permitting increased growth in monogastric meat producing animals which comprises orally administering to such animals an amount of Berninamycin A effective for permitting such increased growth.

0112233

8.   The method of Claim 7 wherein such monogastric meat producing animals are chickens or swine.

9.   The method of Claim 8 wherein Berninamycin A is continuously administered at a rate of from 1 to 100 ppm in the diet.

10.   A method of Claim 9 wherein Berninamycin A is continuously administered at a rate of from 10 to 20 ppm in the diet.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | US-A-3 689 639 (M.E. BERGY et al.)<br>* Claims 1,5; column 8, lines 24-26 *<br><br>--- | 1 | A 23 K    1/17 |
| A | JOURNAL OF THE AMERICAN CHEMICAL SOCIETY, vol. 99, no. 5, 2nd March 1977, pages 1645-1646, Gaston, PA, USA<br>J.M. LIESCH et al.: "Berninamycin. 3. Total structure of berninamycin A" * Page 1645, left-hand column, paragraph 1 *<br><br>----- | 1 | |

|  |  |
|---|---|
|  | TECHNICAL FIELDS SEARCHED (Int. Cl. 3) |
|  | A 23 K<br>A 61 K |

The present search report has been drawn up for all claims

| Place of search | Date of completion of the search | Examiner |
|---|---|---|
| THE HAGUE | 29-02-1984 | DEKEIREL M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82